# FASCICULE DE BREVET EUROPEEN

(11) **EP 1 667 790 B1**
(45) Date de publication et mention de la délivrance du brevet: **16.05.2007**
(21) Numéro de dépôt: 04769494.8
(22) Date de dépôt: 28.09.2004
(51) Int. Cl.: B01F 7/00, B01F 5/04, B01F 3/08, B01F 15/02, B01J 13/02, A61K 9/16

(54) **DISPOSITIF ET PROCEDE POUR LA FABRICATION DE PARTICULES**
VORRICHTUNG UND VERFAHREN ZUR HERSTELLUNG VON PARTIKELN
DEVICE AND METHOD FOR MAKING PARTICLES

(30) Priorité: 01.10.2003 CH 166403
(43) Date de publication de la demande: 14.06.2006
(73) Titulaire: DEBIO RECHERCHE PHARMACEUTIQUE S.A., 1920 Martigny (CH)
(72) Inventeur: CURDY, Catherine, CH-1110 Morges (CH); DUCREY, Bertrand, CH-1920 Martigny (CH); HEIMGARTNER, Frédéric, CH-1844 Villeneuve (CH); PFEFFERLE, François, CH-1971 Grimisuat (CH)
(74) Mandataire: Grosfillier, Philippe
(86) Numéro de dépôt international: PCT/IB2004/003151
(87) Numéro de publication internationale: WO 2005/032703

(56) Documents cités:
- WO-A-93/10665
- WO-A-03/033097
- DE-A- 3 123 743
- US-A- 2 641 453
- US-A- 4 141 957
- US-A- 4 416 548

## Description

L'invention concerne un dispositif pour la fabrication de microparticules ou de nanoparticules ainsi que sa mise en oeuvre dans un procédé de fabrication desdites particules.

Il est connu de réaliser des microparticules et des nanoparticules par la mise en oeuvre de procédés dans lesquels une phase organique est mélangée à une phase aqueuse. La réalisation de nanoparticules et de microparticules industriellement, pose un problème du fait de leur petite taille. Différents procédés et mises en oeuvre de dispositifs pour la réalisation de microparticules et de nanoparticules sont décrits dans la littérature. Il est notamment connu d'utiliser des techniques telles que la nébulisation dans un courant d'air chaud (spray -drying) ou froid (spray - cooling), la séparation de phase, l'émulsion - extraction de solvant, l'émulsion -évaporation de solvant ou encore des fluides supercritiques.

Par ailleurs, avec les techniques couramment utilisées, il est notamment difficile d'obtenir des particules de formes régulières et de tailles homogènes. De plus, des difficultés lors de l'étape d'isolation de telles particules, notamment, par filtration ou par tamisage, ne permettent pas d'optimiser le rendement de fabrication.

EP 0471 036 décrit un procédé de fabrication de nanoparticules et de microparticules dans lequel, dans un homogénéisateur de type Silverson, une phase organique est dispersée dans un milieu saturé en solvant identique à celui contenu dans ladite phase organique, de manière à former une première émulsion huile dans l'eau. Cette émulsion, constituée de micro-gouttes, est transférée aussi rapidement que possible dans un milieu permettant d'extraire 20 à 30% du solvant contenu dans les micro-gouttes. Cette seconde étape permet d'obtenir des microparticules et nanoparticules durcies. Par ailleurs, le problème rencontré avec ce procédé est qu'il faut adapter l'équipement en fonction des quantités de la phase organique de départ.

WO 98/35654 décrit un procédé en continu pour la fabrication de microparticules. Avec le dispositif utilisé, le procédé ne permet pas d'obtenir des particules de taille définie et de forme régulière. En effet, le positionnement des entrées des phases et celui de la sortie du compartiment d'homogénéisation sont tels qu'un volume d'air occupe en partie le compartiment d'homogénéisation tout au long de la phase d'homogénéisation et de ce fait des turbulences se créent dans le compartiment entraînant la formation de particules de forme irrégulières.

WO 03/033097 concerne l'utilisation d'un appareil rotor/stator pour la fabrication de fines particules par mode de précipitation ou de cristallisation. Dans le dispositif de cet art antérieur il n'est pas fait usage d'une canule et encore moins d'un moyen d'entrée perforé pour une meilleure diffusion des phases dans la chambre d'homogénéisation. Par ailleurs, le rotor utilisé est un rotor avec des parois ayant une structure type « trémie ». De plus, les phases mises en jeu subissent des forces de brassage pour leur mélange.
Enfin, il est à noter que le positionnement de la sortie est telle qu'un vide se forme inévitablement ce qui ne favorise pas la formation de petites particules de forme régulière.

Le document US-A-4 416 548 décrit un dispositif comprenant les caractéristiques du préambule de la revendication 1.

Dans le dispositif selon la présente invention du fait du positionnement perpendiculaire des dents du rotor / stator, les deux phases passent à travers les dents et ce sont les forces de cisaillement qui contribuent à une bonne homogénéisation du mélange et la réalisation de particules régulières de petite taille.
Les problèmes rencontrés par le passé ont pu être résolus par le dispositif selon l'invention et sa mise en oeuvre dans un procédé en continu pour la fabrication de nanoparticules ou de microparticules selon l'invention.

L'un des buts de la présente invention est donc de proposer un dispositif selon la revendication 1 permettant un contrôle de la taille du produit fini et permettant, ainsi, de réaliser en continu aussi bien des nanoparticules que des microparticules de forme régulière.

Un autre but de la présente invention est d'optimiser un procédé de fabrication de nanoparticules ou de microparticules en mettant en oeuvre le dispositif selon l'invention de manière à réaliser rapidement et en continu des nanoparticules ou des microparticules de forme régulière, solides et individualisées.

Un des objets de la présente invention est un dispositif comprenant un compartiment d'homogénéisation, comprenant lui-même un système de remplissage, un système de rotor / stator et un moyen de sortie, pour la fabrication en continu de microparticules ou de nanoparticules à partir, au moins, d'une phase aqueuse et d'une phase organique.

Un autre objet de la présente invention est un procédé de fabrication de microparticules ou de nanoparticules mettant en oeuvre ledit dispositif selon la revendication 8, et un procédé de fabrication en continu de microparticules ou de nanoparticules selon la revendication 12.

De plus, dans la suite de la description, on emploiera l'expression « un système de rotor / stator » pour désigner un système constitué d'une pièce fixe, « le stator », dans laquelle est emboîtée une pièce mobile, « le rotor ». Dans un mode particulier de l'invention, « le rotor » et « le stator » sont munis de dents permettant, par rotation, le mélange de différentes substances et leur homogénéisation.

On emploiera l'expression « dents du rotor » pour désigner les parties saillantes du rotor et du stator.

On emploiera, dans la suite de la description, l'expression « compartiment d'homogénéisation » pour désigner l'enceinte fermée dans laquelle les phases sont mises en contact et se mélangent de manière homogène.

Dans la suite de la description, on utilisera l'expression « perforations » pour désigner des trous de taille inférieure ou égale 1 mm.

Dans la suite de la description, on emploiera l'expression « canule » pour désigner un conduit permettant le passage de substances.

On emploiera, dans la suite du texte, l'expression « substance active » pour désigner une substance ayant au moins une caractéristique pharmaceutique.

On emploiera, dans la suite du texte, l'expression « solvant » pour désigner le milieu organique dans lequel on solubilise un ou plusieurs polymères.

On emploiera l'expression « polymère », dans la suite du texte, pour désigner la matrice constituées d'unités polymérisées jouant le rôle d'agent contrôlant la libération des substances actives.

On emploiera l'expression « récipient tampon » pour désigner le récipient placé en sortie du compartiment d'homogénéisation dans le but de recueillir un volume suffisant de suspension de particules, permettant d'amorcer une pompe pour la filtration ou l'ultrafiltration des particules.

On emploiera l'expression « phase aqueuse » pour désigner la phase externe composée au moins d'eau et de surfactant permettant l'extraction du solvant organique et le durcissement des particules.

On emploiera l'expression « surfactant » ou « tensio actif » pour désigner la substance ajoutée dans la phase aqueuse permettant de stabiliser l'émulsion.

On emploiera l'expression « phase organique » pour désigner la solution ou la suspension ou l'émulsion comprenant au moins un polymère et une substance active.

La présente invention concerne un dispositif pour la fabrication en continu de microparticules ou de nanoparticules à partir au moins d'une phase aqueuse et d'une phase organique, constitué par un compartiment d'homogénéisation (1) comprenant au moins une entrée (2) pour délivrer la phase organique, une entrée (3) pour délivrer la phase aqueuse, un système mélangeur (4) et une sortie (5), caractérisé en ce que
a) l'entrée (2) est une canule pour délivrer la phase organique et est positionnée co-axialement à l'axe dudit système mélangeur (4),
b) l'embout (6) de ladite canule est dans un volume (A) délimité par le système mélangeur (4) dans le compartiment d'homogénéisation (1),
c) l'embout (7) de l'entrée (3) est dans le volume (B) délimité entre la paroi (8) du compartiment d'homogénéisation (1) et l'extrémité (9) du système mélangeur (4), et
d) la sortie (5) est dans la paroi supérieure du compartiment d'homogénéisation.

Dans le dispositif selon la présente invention, l'entrée (2) et la sortie (5) sont positionnées de telle manière que l'on puisse éviter un excès d'entrée d'air dans le compartiment d'homogénéisation afin d'éviter la formation de particules déformées.
L'entrée (2) est positionnée co-axialement à l'axe du système mélangeur (4), soit dans l'axe dudit système, et la sortie (5) est dans la paroi supérieure du compartiment d'homogénéisation(1).
Dans le dispositif selon la présente invention, l'entrée (2) est une canule pour délivrer la phase organique et l'entrée (3) pour délivrer la phase aqueuse sont disposées de telle manière que ces deux phases sont délivrées simultanément et de manière homogène dans le compartiment d'homogénéisation (1).

De plus, pour favoriser une bonne dispersion de la phase organique dans la phase aqueuse, l'embout (6) de ladite canule est dans un volume (A) délimité par le système mélangeur (4) dans le compartiment d'homogénéisation (1) et l'embout (7) de ladite entrée (3) est dans un volume (B) délimité entre la paroi (8) du compartiment d'homogénéisation (1) et l'extrémité (9) du système mélangeur (4).

De préférence, dans le dispositif selon l'invention, la canule est fermée ou non à son embout (6) et présente des perforations (10), de manière à favoriser une dispersion fine de la phase organique dans la phase aqueuse, dans le compartiment d'homogénéisation (1).

Les perforations (10) se trouvent sur la partie finale de la canule entrant dans le volume (A). Elles peuvent être sur une ou plusieurs rangées ou de manière aléatoire.

Dans un mode de réalisation du dispositif selon l'invention, le nombre de perforations est au minimum de 1 à 5. Dans un mode avantageux de réalisation, le nombre est de 1 à 10 et dans un mode encore plus avantageux, le nombre est de 1 à 20.

Les perforations (10) peuvent être obtenues mécaniquement par perforation de la paroi de la canule à l'aide d'un micro-foret ou d'un laser, par exemple.

On peut également utiliser une canule dont la partie finale se trouvant dans le volume (A), est dans un matériel comme, par exemple, du verre fritté ou de la maille métallique. On a ainsi une canule dont la partie finale a une multitude de perforations (10) pouvant être de dimension inférieure à 0,01 mm.

Les perforations (10) peuvent être de 0,01 mm à 1 mm. De préférence, les perforations (10) sont de 0,01 mm à 0,9 mm et de manière encore plus préférée de 0,01 mm à 0,7 mm. Le choix de la dimension des perforations permet également d'optimiser la dispersion de la phase organique dans la phase aqueuse dans le compartiment d'homogénéisation (1), mais, surtout, d'optimiser l'exactitude de la taille souhaitée des nanoparticules ou des microparticules.

Dans le dispositif selon l'invention, la vitesse tangentielle du système mélangeur (4) est de 1,5 m/s à 50 m/s et de manière préférée de 2,5 m/s à 41 m/ s.

Dans un mode de réalisation de l'invention, le système mélangeur (4) est un rotor (11) / stator (12).

Le système rotor (11) / stator (12) peut comprendre au moins une rangée de dents (13) et l'espacement (14) entre les dents (13) peut être de 1 à 4 mm. Plus l'espacement est petit, plus il est aisé de réaliser des particules de petite taille. A l'inverse, plus l'espacement est grand, plus il est aisé de réaliser des particules de taille plus grande.

De préférence, les dimensions du système de rotor (11) / stator (12) sont telles que ledit système occupe 4% à 40% du volume du compartiment d'homogénéisation (1).

La présente invention a également pour objet un procédé en continu de fabrication de microparticules ou de nanoparticules mettant en oeuvre le dispositif selon l'invention.

Ledit procédé est tel que l'on délivre simultanément une phase organique comprenant au moins une substance active, un polymère et un solvant par l'entrée (2) qui est une canule et une phase aqueuse comprenant au moins un surfactant par l'entrée (3) dans le compartiment d'homogénéisation (1) dans lequel le système mélangeur (4) a une vitesse tangentielle de 1,5 m/s à 50 m/s permettant simultanément la formation d'une émulsion desdites phases et l'extraction du solvant contenu dans la phase organique, de manière à obtenir une suspension de particules à partir de laquelle on isole les nanoparticules ou les microparticules.

De préférence, dans le procédé selon l'invention mettant en oeuvre ledit dispositif, la vitesse tangentielle du système mélangeur (4) est de 1,5 m/s à 50 m/ s et au moins de 2,5 m/ à 41 m/s.

De préférence, dans le procédé selon l'invention, le système mélangeur (4) est un système rotor (11) / stator (12).

Dans un mode préféré du procédé selon l'invention, on délivre la phase organique par la canule qui est fermée ou non à son embout (6) et qui présente des perforations (10), de manière à disperser de manière radiale ladite phase dans la phase aqueuse dans le compartiment d'homogénéisation (1).

Pour isoler les nanoparticules ou les microparticules à partir de la suspension de particules, on peut évacuer ladite suspension par la sortie (5) du compartiment d'homogénéisation (1) puis effectuer une filtration directe ou tangentielle. On peut également effectuer une sédimentation simple ou forcée à l'aide d'un dispositif de type centrifugation en continu ou essoreuse, après évacuation de ladite suspension par la sortie (5) du compartiment d'homogénéisation (1).

Dans un mode réalisation du procédé selon l'invention pour la fabrication de nanoparticules, on isole les nanoparticules à partir de la suspension de particules en évacuant ladite suspension par la sortie (5) du compartiment d'homogénéisation (1) dans un récipient tampon puis on effectue en continu une ultrafiltration de ladite suspension.

Dans un autre mode réalisation du procédé selon l'invention pour la fabrication de microparticules, on isole les microparticules à partir de la suspension de particules en évacuant ladite suspension par la sortie (5) du compartiment d'homogénéisation (1) dans un récipient tampon puis on effectue en continu une filtration de ladite suspension.

Dans le procédé selon l'invention, la phase organique peut comprendre 1 à 30% de polymère dans au moins un solvant, mais au moins 2 à 25% et en tous les cas 5 à 20 %.

Le mélange polymère-substance active présent dans la phase organique peut comprendre 0,1 à 50% de substance active mais au moins 0,5 à 40% de substance active et en tous les cas 1 à 30% de substance active.

Dans le procédé selon la présente invention mettant en oeuvre ledit dispositif, la phase organique peut être sous forme de solution, d'émulsion ou de suspension.

La phase organique est sous forme de solution lorsque la substance active est solubilisée avec les autres composés de la phase organique.

La phase organique est sous forme d'émulsion lorsque la substance active est solubilisée dans de l'eau puis émulsionnée avec les autres composés de la phase organique.

Enfin la phase organique est sous forme de suspension lorsque la substance active n'est pas solubilisée et qu'elle se trouve sous forme dispersée dans la phase organique.

La substance active hydrosoluble peut notamment être un peptide, un polypeptide, une protéine ou respectivement un sel acceptable d'un point de vue pharmaceutique.

Selon la présente invention, la substance active peut être la gonadoréline (LHRH) ou un de ses dérivés (agonistes et antagonistes), la thyrotropine (TSH), la protiréline (TRH), l'hormone folliculo-stimulante (FSH), la parathyrine (PTH), l'insuline et autres peptides hypoglycémiants, le C-peptide, les analogues de l'exenatide, les analogues du GLP-1 et autres peptides anti-obésité, les antagonistes du récepteur TCR des lymphocytes, la somatostatine ou un de ses dérivés, la corticotropine (ACTH), une hormone de croissance (GH), la somatoréline (GHRH), le peptide libérant l'hormone de croissance (GHRP), la calcitonine, l'endorphine, un interféron, une interleukine, le facteur de nécrose tumorale (TNF), l'erythropoïétine (EPO), un facteur stimulant une colonie (G-CSF, GM-CSF, M-CSF), un facteur de croissance des nerfs (NGF), une somatomédine (IGF), l'amyline et ses analogues synthétiques, la bone morphogenic protein (BMP), la sérotonine, le GABA, la superoxyde dismutase, un immunomodulateur (EGF, LPS), un anticancéreux tel que l'actinomycine D, le bléomycine, le busulfan, le carboplatine, le cisplatine, l'oxaliplatine, la carmustine, le chlorambucile, la cladribine, la cyclophosphamide, la cytarabine, la dacarbazine, la daunorubicine, la doxorubicine, l'estramustine, l'étoposide, la floxuridine, la fludarabine, la fluorouracile, l'hexamethylmélamine, l'hydroxyurée, l'idarubicine, l'ifosfamide, l'asparaginase, la lomustine, la méchloréthamine, le melphalan, la mercaptopurine, le méthotrexate, la mithramycine, la mitomycine C, la mitotane, la mitozantrone, la pentostatine, la procarbazine, la streptozocine, la teniposide, la thioguanine, la thiopeta, la vinblastine, la vincristine et semblables ; un antiviral ; un analgésique tel que le chlorhydrate de péthidine, le tartrate de lévorphanol, , le chorhydrate de morphine, l'oxymorphone; un antagoniste de narcotiques tels que naloxone, naltrexone ou autres ; un anesthésique local tel que la lidocaïne, la xylocaïne et semblables; la cyclosporine et dérivés ; un antiépileptique; un antidépresseur ; un anticoagulant tel que l'héparine naturelle ou synthétique ; un inhibiteur de l'élastase telle que l'EPI-hNE, particulièrement l'EPI-hNE4; une substance pour le traitement de la dégénérescence rétinienne telle qu'un stéroïde ou autre substance peptidique, un antifongique ; un inhibiteur de la résorption osseuse tel un biphosphonate, l'alendronate, et semblables; un antigène de bactéries, un virus, un antidiabétique tel que le glizipide ; un enzyme ; un acide nucléique ; un neuroleptique antipsychotique tel que l'olanzapine, la rispéridone, et semblables ; un inhibiteur de l'alpha-réductase tel que le finastéride ou le dutastéride ; un inhibiteur de l'aromatase tel que l'anastrosole et l'exémestane; une hormone telle que les thyroxines, les estrogènes ; une substance pour la thérapie hormonale telle que le tamoxifène et le 4-OH tamoxifène; une vitamine ; l'huperzine et ses dérivés

La substance active peut être un sel de peptide. Elle peut être un mono- , di-, ou tri - sel. Selon la présente invention, le sel de peptide peut être un sel formé avec un acide inorganique tel que l'acide chlorhydrique, l'acide sulfurique ou l'acide nitrique, par exemple. Il peut également être un sel formé avec un acide organique, tel que, par exemple, l'acide carbonique, l'acide bicarbonate, l'acide succinique, l'acide acétique, l'acide propionique ou l'acide trifluoroacétique. De préférence, le sel de peptide est un sel formé avec un acide organique et de manière avantageusement préférée l'acide organique est l'acide acétique ou l'acide pamoïque.

La substance active préférée est l'olanzapine, l'alendronate, le tamoxifène, le 4-OH tamoxifène, et dérivés, les dérivés de la LHRH, en particulier le pamoate de triptoréline, les dérivés de la somatostatine, en particulier le pamoate de vapréotide, héparine naturelle ou synthétique, les neuroleptiques, la PTH, l'insuline et autres peptides hypoglycémiants, le C-peptide, l'exenatide et ses analogues, le GLP-1 et ses analogues, la cyclosporine et ses dérivés, la calcitonine, les interférons, les interleukines, l'EPO, le CSF, l'oxaliplatine, les antidiabétiques tel que le glizipide, les inhibiteurs de l'alpha réductase, la thyroxine, les oestrogènes, l'huperzine et ses dérivés.

Selon la présente invention, le polymère utilisé est de préférence un polymère biodégradable ou biocompatible sélectionné parmi les acides poly-lactique, acides poly-glycoliques, les copolymères d'acides lactique et glycolique, les copolymère d'acide lactique et de caprolactone ou autres polymères biodégradables tels que d'autres polymères aliphatiques, acide poly-citrique, acide poly-malique, poly-succinates, poly-butylsuccinates, poly-fumarates, polyhydroxybutyrates, poly-caprolactones, poly-carbonates, poly-esteramides, poly-anhydrides, poly-amino acides, poly-orthoesters ou leurs co-polymères avec le PEG, poly-alkyl-cyanoacrylates, poly-etheresters, poly-dioxanones, copolymères avec le poly-éthylène glycol tels que par exemple les co-blocks PBS-PEG décrits dans WO 99/55760, les co-blocks PLA-PEG décrits dans US-5,766,635 ou les co-blocks PLGA-PEG, les poly-uréthanes biodégradables et les poly-phosphazènes ou leurs copolymères avec le PEG.
Des polymères non-biodégradables appropriés sont l'acide poly-acrylique, l'acide poly-méthacrylique, les copolymères de l'acide acrylique et de l'acide méthacrylique, l'éthylcellulose, l'acétylcellulose, nitrocellulose, etc. Ces polymères peuvent être des homopolymères ou copolymères de deux monomères ou plus ou encore des mélanges de polymères.

De préférence, le polymère est sélectionné parmi le groupe comprenant les copolymères de l'acide lactique et glycolique, l'acide poly-Iactique, les copolymères d'acide poly-lactique et de caprolactone, les copolymères de poly-éthylène glycol ou poly-propylène glycol avec d'autres groupes tels que PBS-PEG, PLA-PEG ou co-blocks PLGA-PEG, poly-orthoesters et poly-phosphazènes et leurs copolymères avec le PEG.

Selon la présente invention, le solvant organique utilisé est choisi parmi les solvants non-miscibles ou peu miscibles à l'eau comme les esters tel que l'acétate d'éthyle, l'acétate de butyle, les hydrocarbures halogénés tel que le dichlorométhane, le chloroforme, le chloroéthane, le dichloroéthane, le trichloroéthane, les éthers tel que l'éther éthylique, l'éther isopropylique, les hydrocarbures aromatiques tel que le toluène, le xylène, les carbonates tel que le diethyl carbonate ou semblable.

De préférence, le solvant utilisé est un ester ou un hydrocarbure halogéné.

De préférence, le solvant utilisé est l'açétate d'éthyle.

Des solvants miscibles à l'eau tels que l'éthanol, le diméthylformamide le diméthylsulfoxyde, les pyrrolidones substituées comme le N-méthyl pyrrolidone ou le propylène glycol peuvent être ajoutés aux solvants non miscibles à l'eau.

Dans un mode de réalisation du procédé selon l'invention, on peut utiliser les solvants mentionnés ci-dessus seuls ou mélangés.

Dans un mode préféré du procédé selon la présente invention, la phase organique comprend au moins
- comme substance active l'olanzapine, l'alendronate, le tamoxifène, le 4-OH tamoxifène, et dérivés, les dérivés de la LHRH, en particulier le pamoate de triptoréline, les dérivés de la somatostatine, en particulier le pamoate de vapréotide, héparine naturelle ou synthétique, les neuroleptiques, la PTH, l'insuline et autres peptides hypoglycémiants, la calcitonine, les interférons, les interleukines, l'EPO, le CSF, l'oxaliplatine, les antidiabétiques tels que le glizipide, les inhibiteurs de l'alpha réductase, la thyroxine, les oestrogènes, l'huperzine et ses dérivés,
- comme polymère, un copolymère de l'acide lactique et glycolique, l'acide poly-lactique, un copolymère d'acide poly-lactique et de caprolactone, un copolymère de poly-éthylène glycol ou un poly-propylène glycol avec d'autres groupes tels que PBS-PEG, PLA-PEG ou co-blocks PLGA-PEG, poly-orthoesters et poly-phosphazènes et leurs copolymères avec le PEG, et
- comme solvant l'acétate d'éthyle.

Dans le procédé selon l'invention, la phase aqueuse peut comprendre 0,05 à 5% de tensio-actif et, au moins, 0,1 à 2%.

Selon la présente invention les tensio-actifs utilisés sont le polyvinyl pyrrolidone, l'alcool polyvinylique, le carboxyméthylcellulose, la lécithine ou la gélatine, des tensio-actifs anioniques tel que l'oléate de sodium, le stéarate de sodium ou le lauryl sulfate de sodium, des tensio-actifs non ioniques tels que les esters de sorbitane polyoxyéthylènés ou un dérivé d'huile de castor polyoxyéthylènée.

De préférence, le tensio-actif utilisé est l'alcool polyvinylique.

Les exemples ci-dessous sont là pour illustrer l'invention mais ne sont pas limitatifs.

Les dimensions des microparticules sont mesurées par granulométrie au laser à l'aide d'un appareil, le Mastersizer® (Malvern Instruments) et les dimensions des nanoparticules sont mesurées à l'aide d'un appareil, le Zetasizer® (Malvern Instruments).

Pour la préparation de la phase organique, on utilise un homogénéisateur tel que le Polytron PT 3000 /PT 3100.

Le taux d'encapsulation est mesuré par une méthode analytique appropriée, par exemple par méthode HPLC-UV suite à une extraction dans du phosphate de triéthanolamine (TEAP) pour les peptides, ou encore par spectrophotométrie UV-visible après solubilisation complète dans le diméthyl formamide (DMF) pour l'olanzapine.

Le rendement d'encapsulation exprimé en % correspond au ratio entre le taux d'encapsulation mesuré et le taux d'encapsulation théorique.

La description de la présente invention est faite en référence aux dessins sur lesquels :

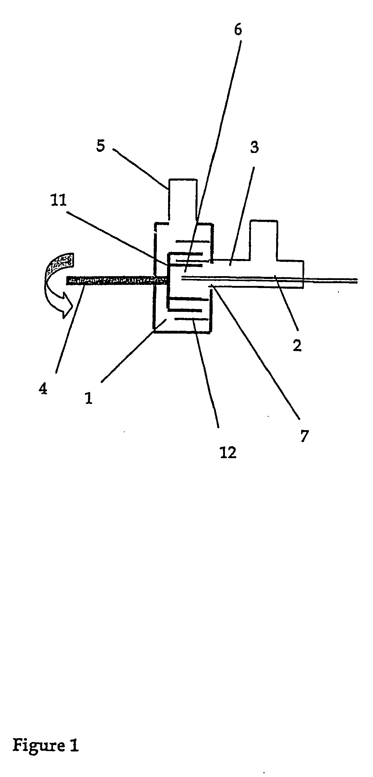

**La figure 1** est une représentation schématique du dispositif selon la présente invention pour la fabrication de microparticules ou de nanoparticules,

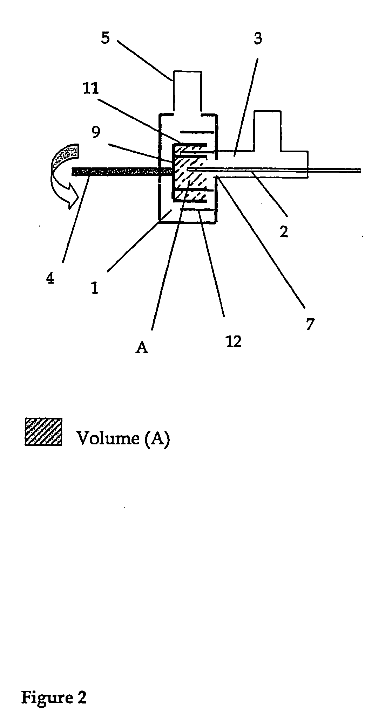

**la figure 2** est une représentation schématique du volume (A) du dispositif selon la présente invention pour la fabrication de microparticules ou de nanoparticules,

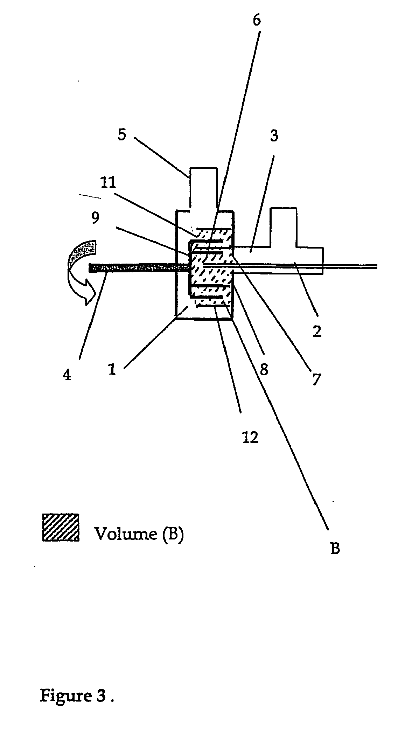

**la figure 3** est une représentation schématique du volume (B) délimité entre la paroi (8) du compartiment d'homogénéisation (1) et l'extrémité (9) du système mélangeur (4) dans le dispositif selon la présente invention pour la fabrication de microparticules ou de nanoparticules,

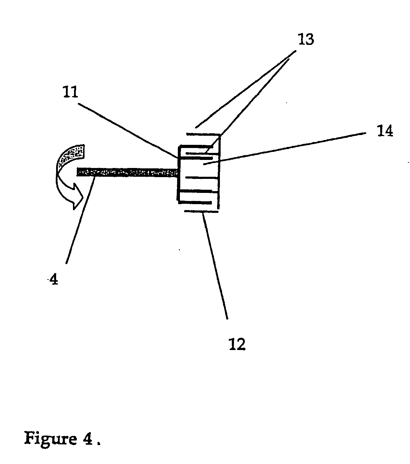

**la figure 4** est une représentation schématique du système mélangeur (4) constitué par un rotor (11) / stator (12) et compris dans la chambre d'homogénéisation (1),

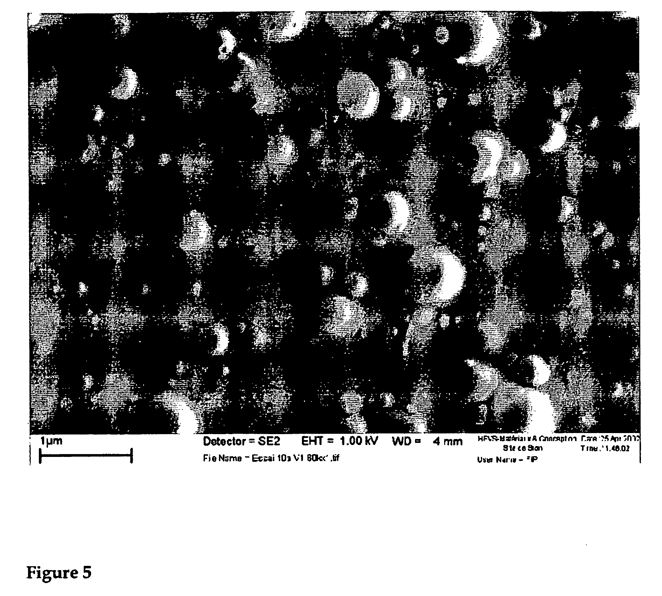

**la figure 5** est une photo de nanoparticules fabriquées selon la mise en oeuvre du procédé selon la présente invention,

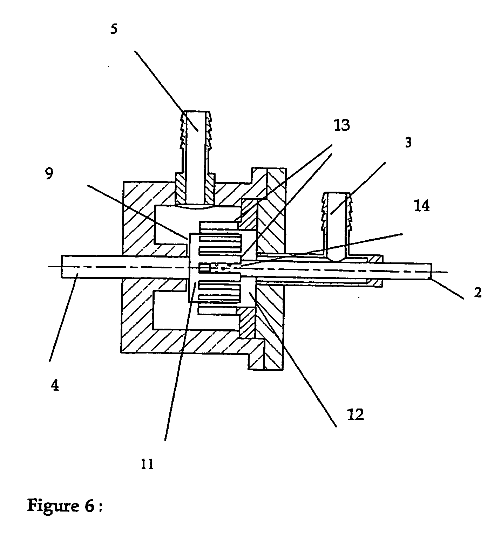

**la figure 6** est également une représentation schématique du système mélangeur (4) constitué par un rotor (11) / stator (12) et compris dans la chambre d'homogénéisation (1),

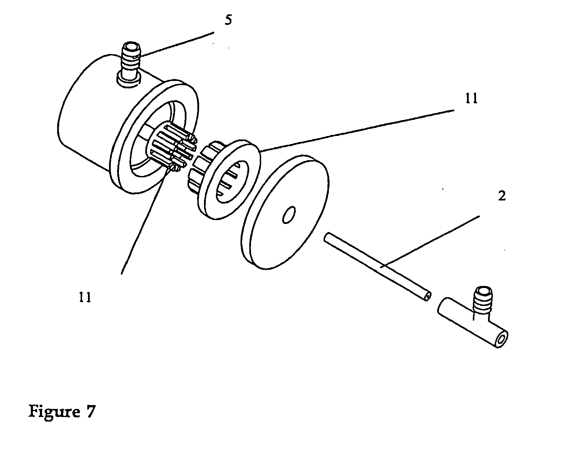

**la figure 7** est une représentation schématique du rotor (11) / stator (12) compris dans la chambre d'homogénéisation (1), des entrées (2) et (3) et de la sortie (5), et

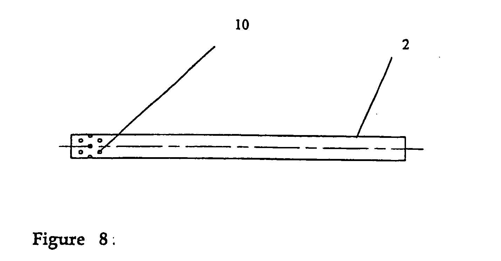

**la figure 8** est une représentation schématique d'une canule présentant des perforations (10).

### Exemple 1

On prépare des microparticules d'acétate de vapréotide dans du PLGA 50/50 de faible poids moléculaire.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 D,L lactide-co-glycolide (PLGA) dans 8 g d'acétate d'éthyle sous agitation magnétique. Le polymère PLGA présente une viscosité inhérente de 0,17 dl/g correspondant à un poids moléculaire moyen de 10 000.
On dissout 329 mg d'acétate de vapréotide sous agitation magnétique dans 800 µl de DMSO (diméthylsulfoxyde) puis on incorpore cette solution à la phase organique ci-dessus. On obtient une solution (phase organique) homogène.

On utilise le dispositif selon l'invention.

On délivre simultanément dans le compartiment d'homogénéisation (1) par l'entrée (2), la canule, à un débit de 10 g/min, 11,1 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'acétate de vapréotide par filtration sur membrane 1,2 µm.

Les particules sont lavées avec de l'eau purifiée.

On peut ensuite congeler et lyophiliser lesdites particules.

Le taux d'encapsulation mesuré est de 9,4% ce qui correspond à un rendement d'encapsulation d'environ 75%. Le diamètre moyen des particules est de 25 µm.

### Exemple 2

On prépare des microparticules d'acétate de vapréotide dans du PLGA 50/50 de poids moléculaire de 35 000 et ayant une viscosité inhérente 0,34 dl/ g.

Pour cela, on prépare la phase aqueuse et la phase organique comme mentionné ci-dessus à l'exemple 1.

On utilise le dispositif selon l'invention.

On délivre simultanément dans le compartiment d'homogénéisation (1) par l'entrée (2), la canule, à un débit de 10 g/min,11,1 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'acétate de vapréotide par filtration sur membrane 1,2 µm.

Les particules sont lavées avec de l'eau purifiée.

On peut ensuite congeler et lyophiliser lesdites particules.

Le taux d'encapsulation mesuré est de 8,5% ce qui correspond à un rendement d'encapsulation d'environ 68%. Le diamètre moyen des particules est de 30 µm.

### Exemple 3

On prépare des microparticules de pamoate de vapréotide dans du PLGA 50/50 de poids moléculaire de 35 000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 4 g de polymère 50/50 poly(D,L lactide-co-glycolide) (PLGA) dans 8 g d' acétate d'éthyle sous agitation magnétique.
On suspend 700 mg de pamoate de vapréotide dans 8 g d'acétate d'éthyle au polytron à 20000 rpm pendant 3 minutes puis on incorpore cette suspension à la solution organique ci-dessus et l'ensemble est homogénéisé au polytron à 3000 rpm pendant 20 secondes.

On utilise le dispositif selon l'invention.

On délivre simultanément dans le compartiment d'homogénéisation (1) par l'entrée (2), la canule, à un débit de 10 g/min, 20,7 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'acétate de vapréotide par filtration sur membrane 1,2 µm.

Les particules sont lavées avec de l'eau purifiée.

On peut ensuite congeler et lyophiliser lesdites particules.

Le taux d'encapsulation mesuré est de 10% ce qui correspond à un rendement d'encapsulation d'environ 96%. Le diamètre moyen des particules est de 32 µm.

### Exemple 4

On prépare des microparticules d'olanzapine dans du PLGA 50/50 de poids moléculaire de 35 000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 poly(D,L lactide-co-glycolide) (PLGA) dans 8 g d'acétate d'éthyle sous agitation magnétique.

On dissout 225 mg d'olanzapine dans la phase organique

On utilise le dispositif selon l'invention.

On délivre simultanément dans le compartiment d'homogénéisation (1) par l'entrée (2), la canule, à un débit de 10 g/min,10,2 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'olanzapine par filtration sur membrane 1,2 µm.

Les particules sont lavées avec de l'eau purifiée.

On peut ensuite congeler et lyophiliser lesdites particules.

Le taux d'encapsulation mesuré est de 6,9% ce qui correspond à un rendement d'encapsulation d'environ 68%. Le diamètre moyen des particules est de 44 µm.

### Exemple 5

On prépare des microparticules d'acétate de triptoréline dans du PLGA 50/50 de poids moléculaire de 35 000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 poly(D,L lactide-co-glycolide) (PLGA) dans 4 g d'acétate d'éthyle sous agitation magnétique.

On suspend 200 mg d'acétate de triptoréline au polytron à 20000 rpm dans 4 g d'acétate d'éthyle puis on incorpore cette suspension à la phase organique ci-dessus. L'ensemble est homogénéisé au Polytron à 3000 rpm

On utilise le dispositif selon l'invention.

On délivre simultanément dans le compartiment d'homogénéisation (1)par l'entrée (2) qui est une canule, à un débit de 10 g/min,10,2 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'acétate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 8,9% ce qui correspond à un rendement d'encapsulation d'environ 100%. Le diamètre moyen des particules est de 45 µm.

### Exemple 6

On prépare des microparticules de calcitonine de saumon dans du PLGA 50/50 de poids moléculaire moyen 35000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 poly(D,L lactide-co-glycolide)(PLGA) dans 4 g d'acétate d'éthyle sous agitation magnétique.

On suspend 200 mg de calcitonine de saumon dans 4 g d' acétate d'éthyle à l'aide d'un Polytron à 20000 rpm puis on incorpore cette suspension à la phase organique ci-dessus. L'ensemble est homogénéisé au Polytron à 3000 rpm pendant 20 secondes.

On utilise le dispositif selon l'invention.

On délivre simultanément dans le compartiment d'homogénéisation (1) par l'entrée («2) qui est une canule, à un débit de 10 g/min,11,1 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/ s soit 4000 rpm.

On obtient ainsi une suspension de particules à partir de laquelle on isole les microparticules de calcitonine de saumon par filtration sur membrane 1,2 µm.

Les particules sont lavées avec de l'eau purifiée.

On peut ensuite congeler et lyophiliser lesdites particules.

Le diamètre moyen des particules est de 40 µm.

### Exemple 7

On prépare des microparticules d'alendronate de sodium dans du PLGA 50/50 de poids moléculaire moyen environ 35000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 4 g de polymère 50/50 poly (D,L lactide-co-glycolide) (PLGA) dans 8 g d'acétate d'éthyle sous agitation magnétique.

On suspend 200 mg d'alendronate de sodium dans 8 g d'acétate d'éthyle à l'aide d'un Polytron à 20000 rpm puis on incorpore cette suspension à la phase organique ci-dessus. L'ensemble est homogénéisé au Polytron à 3000 rpm pendant 20 secondes.

On utilise le dispositif selon l'invention.

On délivre simultanément dans le compartiment d'homogénéisation (1) par l'entrée (2) qui est une canule, à un débit de 10 g/min, 11,1 g de la phase organique telle que préparée ci-dessus et par l'entrée (3), à un débit de 150 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules de calcitonine de saumon par filtration sur membrane 1,2 µm.

Les particules sont lavées avec de l'eau purifiée.

On peut ensuite congeler et lyophiliser lesdites particules.

Le diamètre moyen des particules est de 46 µm.

### Exemple 8

On prépare des microparticules d'acétate de triptoréline dans du PLGA 50/50 de poids moléculaire environ 35000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 poly(D,L lactide-co-glycolide) (PLGA) dans 8 g d'acétate d'éthyle sous agitation magnétique.

On dissout 100 mg d'acétate de triptoréline dans 1,3 g de solution de Tween 80 à 20% puis on émulsionne cette solution dans la phase organique ci-dessus à l'aide du Polytron à 15000 rpm pendant 3 minutes.

On utilise le dispositif selon l'invention.

On délivre simultanément dans le compartiment d'homogénéisation (1) par la canule (2), à un débit de 10 g/min,10,1 g de la phase organique telle que préparée ci-dessus et par l'entrée (3), à un débit de 150 ml/ min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les microparticules d'acétate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 5,8% ce qui correspond à un rendement d'encapsulation d'environ 100%. Le diamètre moyen des particules est de 19 µm.

### Exemple 9

On prépare des microparticules de pamoate de triptoréline dans du PLGA 85/15 d'un poids moléculaire moyen d'environ 74 000.

Pour cela, on prépare la phase aqueuse en mélangeant, sous agitation magnétique à une température de 40°C,100 g d'alcool polyvinylique et 4900 g d'eau.

Parallèlement on prépare la phase organique en dissolvant totalement 4 g de polymère 85/15 poly(D,L lactide-co-glycolide) (PLGA) dans 15 g de dichlorométhane sous agitation magnétique.

On suspend 1000 mg de pamoate de triptoréline dans 10 g de dichlorométhane sous agitation magnétique puis on incorpore cette solution à la phase organique ci-dessus.

On utilise le dispositif selon l'invention.

On délivre simultanément dans le compartiment d'homogénéisation (1) par la canule (2), à un débit de 5 ml/min, 30 g de la phase organique telle que préparée ci-dessus et par l'entrée (3), à un débit de 850 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 12,54% ce qui correspond à un rendement d'encapsulation d'environ 90%. Le diamètre moyen des particules est de 70 µm.

### Exemple 10

On prépare des nanoparticules d'olanzapine dans du PLGA 50/50 de poids moléculaire de 35000.

Pour cela, on prépare la phase aqueuse en mélangeant, sous agitation magnétique à une température de 40°C, 5 g d'alcool polyvinylique et 245 g d'eau.

Parallèlement on prépare la phase organique en dissolvant totalement 2 g de polymère 50/50 poly(D,L lactide-co-glycolide) (PLGA) dans 8 g d'acétate d'éthyle sous agitation magnétique.

Puis on dissout 225 mg d'olanzapine dans la phase organique ci-dessus.

On utilise le dispositif selon l'invention.

On délivre simultanément dans le compartiment d'homogénéisation (1) par la canule (2), à un débit de 10 g/min,10,2 g de la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 40 m/s soit 30000 rpm.

On obtient ainsi une suspension de particules à partir de laquelle on isole les nanoparticules d'olanzapine par centrifugation et filtration.

On peut ensuite congeler et lyophiliser lesdites particules.

Le taux d'encapsulation mesuré est de 3,3% ce qui correspond à un rendement d'encapsulation d'environ 33%. La moyenne des particules mesurées est de 230 nm.

### Exemple 11

On prépare des nanoparticules d'olanzapine dans du PBS-PEG de poids moléculaire d'environ 30 000.

Pour cela, on prépare la phase aqueuse en mélangeant, sous agitation magnétique à une température de 40°C, 40 g d'alcool polyvinylique et 1200 g d'eau.

Parallèlement on prépare la phase organique en dissolvant totalement 0,9 g de polymère PBS-PEG ayant une viscosité de 0,64 dl/ g (tel que réalisé dans l'exemple 10 de la demande de brevet WO 99/55760) et 100 mg d'olanzapine dans 19 g de dichlorométhane sous agitation magnétique.

On utilise le dispositif selon l'invention.

On délivre simultanément dans le compartiment d'homogénéisation (1) par la canule (2), à un débit de 10,9ml/min, 22g de phase organique telle que préparée ci-dessus et par l'entrée (3), à un débit de 400 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 40 m/s soit 30 000 rpm.

On obtient ainsi une suspension de particules à partir de laquelle on isole les nanoparticules d'olanzapine par filtration sur 0,22 µm.

Le taux d'encapsulation mesuré est de 3% ce qui correspond à un rendement d'encapsulation d'environ 30%. La moyenne des particules mesurées est de 50 nm.

### Exemple 12

On prépare des microparticules de pamoate de triptoréline dans du PLGA 85/15 d'un poids moléculaire de 74000.

Pour cela, on prépare la phase aqueuse en mélangeant, sous agitation magnétique à une température de 40°C,100 g d'alcool polyvinylique et 4900 g d'eau.

Parallèlement on prépare la phase organique en dissolvant totalement 4 g de polymère 85/15 poly(D,L lactide-co-glycolide) (PLGA) dans 15 g d'acétate d'éthyle sous agitation magnétique.
On disperse au Polytron (20000rpm, 6 minutes) 1000 mg de pamoate de triptoréline dans 10 g d' acétate d'éthyle puis on incorpore cette suspension à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 11,27% ce qui correspond à un rendement d'encapsulation d'environ 80%. Le diamètre moyen des particules est de 33,9 µm.

### Exemple 13

On prépare des microparticules de pamoate de triptoréline dans du PLGA 85/15 d'un poids moléculaire de 74 000.

Pour cela, on prépare la phase aqueuse en mélangeant, sous agitation magnétique à une température de 40°C,10 g d'alcool polyvinylique et 1990 g d'eau.

Parallèlement on prépare la phase organique en dissolvant totalement 4 g de polymère 85/15 poly(D,L lactide-co-glycolide) (PLGA) dans 15 g d'acétate d'éthyle sous agitation magnétique.

On suspend 1000 mg de pamoate de triptoréline dans 10 g d'acétate d'éthyle au Polytron 20000 rpm, 6 minutes, puis on incorpore cette suspension à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 12,4% ce qui correspond à un rendement d'encapsulation d'environ 89%. Le diamètre moyen des particules est de 46,2 µm.

### Exemple 14

On prépare des microparticules de pamoate de triptoréline dans du PLGA 90/10 d'un poids moléculaire environ 30000.

Pour cela, on prépare la phase aqueuse en mélangeant, sous agitation magnétique à une température de 40°C, 40 g d'alcool polyvinylique et 1960 g d'eau purifiée.

Parallèlement on prépare la phase organique en dissolvant totalement 4 g de polymère 90/10 poly (D,L lactide-co-glycolide) (PLGA) dans 15 g d'acétate d'éthyle sous agitation magnétique.
On suspend 1000 mg de pamoate de triptoréline dans 10 g d'acétate d'éthyle au polytron 20000 rpm, 6 minutes, puis on incorpore cette solution à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 10,5% ce qui correspond à un rendement d'encapsulation d'environ 75%. Le diamètre moyen des particules est de 20,7 µm.

### Exemple 15

On prépare des microparticules de pamoate de triptoréline dans du PLA de poids moléculaire environ 30 000.

Pour cela, on prépare la phase aqueuse en mélangeant, sous agitation magnétique à une température de 40°C, 40 g d'alcool polyvinylique et 1960 g d'eau.

Parallèlement on prépare la phase organique en dissolvant totalement 4 g de polymère poly(D,L lactide)- (PLA)dans 15 g d'acétate d'éthyle sous agitation magnétique.
On suspend 1000 mg de pamoate de triptoréline dans 10 g d'acétate d'éthyle au polytron 20000 rpm, 6 minutes, puis on incorpore cette solution à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 10% ce qui correspond à un rendement d'encapsulation d'environ 71%. Le diamètre moyen des particules est de 21,6 µm.

### Exemple 16

On prépare des microparticules de pamoate de triptoréline dans du PLA d'un poids moléculaire environ 70 000.

Pour cela, on prépare la phase aqueuse en mélangeant, sous agitation magnétique à une température de 40°C, 40 g d'alcool polyvinylique et 1960 g d'eau.

Parallèlement on prépare la phase organique en dissolvant totalement 4 g de polymère poly(D,L lactide)- (PLA) dans 15 g d'acétate d'éthyle sous agitation magnétique.

On suspend 1000 mg de pamoate de triptoréline dans 10 g d'acétate d'éthyle au Polytron à 20000 rpm, 6 minutes, puis on incorpore cette suspension à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 11,5% ce qui correspond à un rendement d'encapsulation d'environ 82%. Le diamètre moyen des particules est de 32,1 µm.

### Exemple 17

On prépare des microparticules de pamoate de triptoréline dans du PLA d'un poids moléculaire environ 20 000.

Pour cela, on prépare la phase aqueuse en mélangeant, sous agitation magnétique à une température de 40°C, 40 g d'alcool polyvinylique et 1960 g d'eau.

Parallèlement on prépare la phase organique en dissolvant totalement 4 g de polymère poly(D,L lactide)-(PLA)dans 15 g d'acétate d'éthyle sous agitation magnétique.
On suspend 1000 mg de pamoate de triptoréline dans 10 g d'acétate d'éthyle au Polytron à 20000 rpm, 6 minutes, puis on incorpore cette suspension à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.

On peut ensuite congeler et lyophiliser lesdites particules.

Le taux d'encapsulation mesuré est de 8,83% ce qui correspond à un rendement d'encapsulation d'environ 63%. Le diamètre moyen des particules est de 22,2 µm.

### Exemple 18

On prépare des microparticules de pamoate de triptoréline dans un co-polymère 75/25 poly(D,L-lactide-co-ε-caprolactone) (PLA-PCL) d'un poids moléculaire de 80 000.

Pour cela, on prépare la phase aqueuse en mélangeant, sous agitation magnétique à une température de 40°C, 40 g d'alcool polyvinylique et 1960 g d'eau.

Parallèlement on prépare la phase organique en dissolvant totalement 4 g de polymère co-polymère 75/25 poly(D,L-lactide-co-ε-caprolactone) (PLA-PCL)dans 15 g d'acétate d'éthyle sous agitation magnétique.
On suspend 1000 mg de pamoate de triptoréline dans 10 g d'acétate d'éthyle puis on incorpore cette suspension à la phase organique ci-dessus.

On utilise le dispositif selon l'invention.

On délivre simultanément dans le compartiment d'homogénéisation (1) par la canule (2), à un débit de 5 ml/min, la phase organique telle que préparée ci-dessus et par l'entrée (3), à un débit de 200 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules de pamoate de triptoréline par filtration sur membrane 1,2 µm.

Les particules sont lavées avec de l'eau purifiée.
On peut ensuite congeler et lyophiliser lesdites particules.

Le diamètre moyen des particules est de 35,2 µm.

### Exemple 19

On prépare des microparticules d'héparine de bas poids moléculaire.

Pour cela, on prépare la phase aqueuse en mélangeant, sous agitation magnétique à une température de 40°C, 260 g d'alcool polyvinylique et 12740 g d'H₂O MilliQ.

Parallèlement on prépare la phase organique en dissolvant totalement 4,26 g d'un mélange comprenant 50% de polymère 50/50 poly(D,L lactide-co-glycolide) (PLGA) de viscosité 0,5 dl/g et 50% de polymère Eudragit RS PO dans 83,5 g d' acétate d'éthyle sous agitation magnétique.

On suspend 750 mg de nadroparine dans 16,7 ml d'eau purifiée puis on émulsifie cette solution dans la phase organique à l'aide du Polytron (15000 rpm, 90 secondes).

On utilise le dispositif selon l'invention.

On délivre simultanément dans le compartiment d'homogénéisation (1) par l'entrée (2), la canule, à un débit de 5 ml/min, la phase organique telle que préparée ci-dessus et par l'entrée (3), à un débit de 650 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 3,2 m/s soit 4000 rpm.

On obtient ainsi une suspension de particules à partir de laquelle on isole les particules par filtration sur membrane 1,2 µm.

Les particules sont lavées avec de l'eau purifiée.

On peut ensuite congeler et lyophiliser lesdites particules.

Le diamètre moyen des microparticules est de 23 µm.

### Exemple 20

On prépare des microparticules d'interféron.

Pour cela, on prépare la phase aqueuse en mélangeant, sous agitation magnétique à une température de 40°C,120 g d'alcool polyvinylique et 5880 g d'H₂O MilliQ.

Parallèlement on prépare la phase organique en dissolvant totalement 1,98 g d'un mélange comprenant 50% de polymère 50/50 poly(D,L lactide-co-glycolide) (PLGA) de viscosité 0.5 dl/g et 50% de polymère Eudragit RS PO dans 39 ml d'acétate d'éthyle sous agitation magnétique.

7 ml de la solution d'interféron est préparée en mélangeant 381 µl de la solution d'interféron alpha 17 dans du tampon phosphate pH 8 (1,83 mg protéine / ml), 280 µl de solution d'albumine sérique humaine (50 mg/ml) dans du tampon phosphate pH 8 et 6939 µl de tampon phosphate pH 8.

On émulsifie la solution d'interféron ainsi préparée dans la phase organique à l'aide d'un Polytron (15000 rpm, 90 secondes).

On utilise le dispositif selon l'invention.

On délivre simultanément dans le compartiment d'homogénéisation (1) par l'entrée (2), la canule, à un débit de 5 ml/min, la phase organique telle que préparée ci-dessus et par l'entrée (3), à un débit de 590 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 2,4 m/s soit 3000 rpm.

On obtient ainsi une suspension de particules à partir de laquelle on isole les particules par filtration sur membrane 1,2 µm.

Les particules sont lavées avec de l'eau purifiée.

On peut ensuite lyophiliser lesdites particules.

Le diamètre moyen des microparticules est de 19,1 µm.

### Contre-Exemple 21

On utilise le procédé ainsi qu'un homogénéisateur de type Silverson tels que décrits dans EP 0471 036.

On prépare une phase aqueuse en mélangeant sous agitation magnétique 10 g de PVA (alcool polyvinylique), 0,847 g d'hydrogénophosphate de sodium déshydraté et 489 g de H₂O MilliQ. Enfin on ajoute 39g d'acétate d' éthyle de manière à stabiliser le pH à 8,9.

Parallèlement, on prépare une phase organique en dissolvant 3,4 g de polymère 50/50 poly(D,L lactide-co-glycolide) (PLGA) de viscosité 0,34 dl/g dans 3,4 g d'acétate d'éthyle sous agitation magnétique.
On dissout également 571 mg d'acétate de leuprolide dans 1,549 g de DMSO.

On mélange cette solution contenant l'acétate de leuprolide dans la phase organique sous agitation magnétique.

On pompe la phase organique ainsi préparé dans l'homogénéisateur de type Silverson équipé d'un rotor à 4 pales tournant à 400 rpm.

Parallèlement, on pompe également la phase aqueuse dans cet homogénéisateur à une vitesse de 127 ml/min.

On obtient ainsi une première émulsion, appelée émulsion primaire. On extrait une partie du solvant contenu dans cette émulsion primaire à la sortie de l'homogénéisateur Silverson en pompant de l'eau purifiée à un débit de 1790 ml/min.

On obtient ainsi une suspension de microsphères que l'on collecte dans un récipient contenant 500 ml d'eau purifiée dans lequel on laisse ladite suspension sous agitation magnétique pendant 15 min, de manière à extraire le solvant restant contenu dans cette suspension.

Finalement on filtre la suspension de particules, de manière à obtenir des particules individualisées que l'on lyophilise.

Les particules ainsi obtenues sont de forme allongée et non homogène et le tamisage sur 106 µm est difficile.

### Exemple 22

On prépare des nanoparticules de valérate d'estradiol dans du PLGA 50/50 de poids moléculaire de 35000.

Pour cela, on prépare la phase aqueuse en mélangeant, sous agitation magnétique à température ambiante, 5 g d'alcool polyvinylique et 245 g d'eau

Parallèlement on prépare la phase organique en dissolvant totalement 2.467 g de polymère 50/50 poly(D,L lactide-co-glycolide) (PLGA) dans 50 ml d'acétate d'éthyle sous agitation magnétique.

Puis on dissout 33 mg de valérate d'estradiol dans la phase organique ci-dessus.

On utilise le dispositif selon l'invention.

On délivre simultanément dans le compartiment d'homogénéisation (1) par la canule (2), à un débit de 5 ml/min, la totalité de la phase organique telle que préparée ci-dessus et par l'entrée (3), à un débit de 10 ml/min, la phase aqueuse telle que préparée ci-dessus jusqu'à épuisement de la phase organique.

De manière à former simultanément une émulsion des deux phases et extraire le solvant de la phase organique, on fait tourner le système rotor (11) / stator (12) a une vitesse tangentielle de 26,6 m/s soit 20 000 rpm.

On obtient ainsi une suspension de nanoparticules de valérate d'estradiol de taille moyenne mesurée de 300 nm.

On peut ensuite congeler et lyophiliser lesdites particules.

### Contre-Exemple 23

On utilise le procédé ainsi qu'un homogénéisateur de type Silverson tels que décrits dans le brevet WO 03/033097. On tente de préparer des microparticules de valérate d'estradiol dans du PLGA 75/25.

Pour cela, on prépare la phase aqueuse en mélangeant, sous agitation magnétique à température ambiante, 100 g d'alcool polyvinylique et 4900 g d'eau (2%).

Parallèlement on prépare la phase organique en dissolvant totalement 2,27 g de polymère 75/25 poly(D,L lactide-co-glycolide) (PLGA) dans 25 g d'acétate d'éthyle sous agitation magnétique.

Puis on dissout 229 mg de valérate d'estradiol dans la phase organique ci-dessus.

On utilise le dispositif selon l'invention.

On pompe à un débit de 5 ml/min la phase organique ainsi préparé dans l'homogénéisateur de type Silverson équipé d'un rotor à 4 pales selon le brevet WO 03/033097 tournant à 5500 rpm.

Parallèlement, on pompe également la phase aqueuse dans cet homogénéisateur à une vitesse de 750 ml/min.

On obtient ainsi une suspension de microsphères que l'on collecte dans un récipient sous agitation magnétique.

Par microscopie optique, la suspension contient des microsphères de taille non-homogène ainsi que des filaments.

Finalement la suspension, constituée desdites microparticules et desdits filaments, est filtrée sur filtre 1,2 µm puis lyophilisée.

Les particules obtenues sont de forme filamenteuse et non homogène. La mise en suspension est difficile.

### Exemple 24

Dans cet exemple on utilisera une canule recouverte d'une grille multiperforée à son extrémité.

On prépare des microparticules d'olanzapine dans du PLGA 50/50 de faible poids moléculaire.

Pour cela, on prépare la phase aqueuse en mélangeant, sous agitation magnétique à une température de 40°C, 80 g d'alcool polyvinylique et 3920 g d'eau.

Parallèlement on prépare la phase organique en dissolvant totalement 4,5 g de polymère 50/50 D,L lactide-co-glycolide (PLGA) dans 25 g d'acétate d'éthyle sous agitation magnétique. Le polymère PLGA présente une viscosité inhérente de 0,34 dl/g correspondant à un poids moléculaire moyen de 35000 Da.
On dissout 500 mg d'olanzapine sous agitation magnétique dans la phase organique ci-dessus. On obtient une solution (phase organique) homogène.

On utilise le dispositif selon l'invention. La canule employée est recouverte d'une grille multiperforée à son extrémité.

On délivre simultanément dans le compartiment d'homogénéisation (1) par la canule (2), à un débit de 5 ml/min, 30 g de la phase organique telle que préparée ci-dessus et par l'entrée (6), à un débit de 600 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,0 m/s soit 5000 rpm.

On obtient ainsi une suspension de particules à partir de laquelle on isole les microparticules d'olanzapine par filtration sur membrane 1,2 µm.
Les particules sont lavées avec de l'eau purifiée

On peut ensuite congeler et lyophiliser lesdites particules.

Le taux d'encapsulation mesuré est de 8,6% ce qui correspond à un rendement d'encapsulation d'environ 86%. Le diamètre moyen des particules est de 32 µm.

### Exemple 25

On prépare des microparticules d'estradiol dans du PLGA 50/50 de poids moléculaire de 40000 Da et ayant une viscosité inhérente 0,42 dl/ g.

Pour cela, on prépare la phase aqueuse en mélangeant, sous agitation magnétique à une température de 40°C,160 g d'alcool polyvinylique et 7840 g d'eau.

Parallèlement on prépare la phase organique en dissolvant totalement 4.9 g de polymère 50/50 D,L lactide-co-glycolide (PLGA) dans 50 g d'acétate d'éthyle sous agitation magnétique.
On dissout 100 mg d'estradiol sous agitation magnétique dans 800 µl de DMSO (diméthylsulfoxyde) puis on incorpore cette solution à la phase organique ci-dessus. On obtient une solution (phase organique) homogène.

On utilise le dispositif selon l'invention.

On délivre simultanément dans le compartiment d'homogénéisation (1) par la canule (2), à un débit de 5 ml/min, 55 g de la phase organique telle que préparée ci-dessus et par l'entrée (6), à un débit de 750 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,0 m/s soit 5000 rpm.

On obtient ainsi une suspension de particules à partir de laquelle on isole les microparticules d'estradiol par filtration sur membrane 1,2 µm.
Les particules sont lavées avec de l'eau purifiée.

On peut ensuite congeler et lyophiliser lesdites particules.

Le taux d'encapsulation mesuré est de 1,5% ce qui correspond à un rendement d'encapsulation d'environ 75%. Le diamètre moyen des particules est de 18,9 µm.

### Exemple 26

On prépare des microparticules d'estradiol dans du PLGA 50/50 de poids moléculaire de 50 000 Da et ayant une viscosité inhérente 0,5 dl/ g.

Pour cela, on prépare la phase aqueuse en mélangeant, sous agitation magnétique à une température de 40°C,160 g d'alcool polyvinylique et 7840 g d'eau.

Parallèlement on prépare la phase organique en dissolvant totalement 4.9 g de polymère 50/50 D,L lactide-co-glycolide (PLGA) dans 50 g d'acétate d'éthyle sous agitation magnétique.
On dissout 100 mg d'estradiol sous agitation magnétique dans 800 µl de DMSO (diméthylsulfoxyde) puis on incorpore cette solution à la phase organique ci-dessus. On obtient une solution (phase organique) homogène.

On utilise le dispositif selon l'invention.

On délivre simultanément dans le compartiment d'homogénéisation (1) par la canule (2), à un débit de 5 ml/min, 55 g de la phase organique telle que préparée ci-dessus et par l'entrée (6), à un débit de 750 mi/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,0 m/s soit 5000 rpm.

On obtient ainsi une suspension de particules à partir de laquelle on isole les microparticules d'estradiol par filtration sur membrane 1,2 µm.
Les particules sont lavées avec de l'eau purifiée.

On peut ensuite congeler et lyophiliser lesdites particules.

Le taux d'encapsulation mesuré est de 1,7% ce qui correspond à un rendement d'encapsulation d'environ 85%. Le diamètre moyen des particules est de 23,6 µm.

### Exemple 27

On prépare des microparticules d'estradiol dans du PLGA 75/25 de poids moléculaire de 70 000 Da et ayant une viscosité inhérente 0,65 dl/g.

Pour cela, on prépare la phase aqueuse en mélangeant, sous agitation magnétique à une température de 40°C,160 g d'alcool polyvinylique et 7840 g d'eau.

Parallèlement on prépare la phase organique en dissolvant totalement 4.65 g de polymère 75/25 D,L lactide-co-glycolide (PLGA) dans 50 g d'acétate d'éthyle sous agitation magnétique.
On dissout 350 mg d'estradiol sous agitation magnétique dans 2500 µl de DMSO (diméthylsulfoxyde) puis on incorpore cette solution à la phase organique ci-dessus. On obtient une solution (phase organique) homogène.

On utilise le dispositif selon l'invention.

On délivre simultanément dans le compartiment d'homogénéisation (1) par la canule (2), à un débit de 5 ml/min, 57 g de la phase organique telle que préparée ci-dessus et par l'entrée (6), à un débit de 750 ml/min, la phase aqueuse telle que préparée ci-dessus.

De manière à former simultanément une émulsion des deux phases et extraire le solvant de la phase organique, on fait tourner le système rotor (11) / stator (12) a une vitesse tangentielle de 4,4 m/s soit 5500 rpm.

On obtient ainsi une suspension de particules à partir de laquelle on isole les microparticules d'estradiol par filtration sur membrane 1,2 µm.
Les particules sont lavées avec de l'eau purifiée.

On peut ensuite congeler et lyophiliser lesdites particules.

Le taux d'encapsulation mesuré est de 6% ce qui correspond à un rendement d'encapsulation d'environ 86%. Le diamètre moyendes particules est de 18,4 µm.

## Revendications

1. Dispositif pour la fabrication en continu de microparticules ou de nanoparticules à partir au moins d'une phase aqueuse et d'une phase organique, comprenant un compartiment d'homogénéisation en forme de cylindre (1) qui est défini par une paroi tubulaire formant l'enveloppe dudit cylindre et par une première et une deuxième parois latérales disposées à chaque extrémité de la ladite paroi tubulaire; le dispositif comprenant en outre une première et deuxième entrées (2,3) qui traversent ladite première paroi latérale et qui sont adaptées pour délivrer respectivement une phase organique et une phase aqueuse dans le compartiment d'homogénéisation (1), une sortie (5) adaptée pour extraire du compartiment d'homogénéisation (1) une suspension de particules ; le compartiment d'homogénéisation (1) renfermant un système mélangeur (4) comprenant un ensemble rotor (11)/stator(12), dans lequel
a) lesdites parois latérales sont disposées selon un plan vertical,
b) l'axe de symétrie dudit cylindre est disposé horizontalement,
c) le rotor (11) est monté en rotation autour d'un axe horizontal qui traverse ladite deuxième paroi latérale,
d) ladite première entrée (2) est une canule disposée dans le prolongement de l'axe du rotor (11),
e) le compartiment d'homogénéisation (1) présente un côté supérieur sur lequel se situe ladite sortie (5).
**caractérisé en ce que** ladite canule comprend une partie finale se situant à l'intérieur du rotor et à l'intérieur du stator.

2. Dispositif selon la revendication 1 **caractérisé en ce que** le rotor (11) et le stator (12) sont de forme cylindrique.

3. Dispositif selon la revendication 5, **caractérisé en ce que** le rotor (11) et stator (12) comprennent une rangée de dents (13) et que l'espacement (14) entre les dents (13) est de 1 à 4 mm.

4. Dispositif selon l'une quelconque des revendications précédentes **caractérisé en ce que** la première entrée (2) comporte des perforations (10).

5. Dispositif selon la revendication 4 **caractérisé en ce que** le nombre de perforations (10) est de 1 à 20.

6. Dispositif selon la revendication 4 ou 5 **caractérisé en ce que** les perforations (10) ont un diamètre de 0,01 mm à 1 mm.

7. Dispositif selon l'une quelconque des revendications précédentes **caractérisé en ce que** les dimensions de l'ensemble rotor (11) /stator (12) sont telles que ledit système occupe 4% à 40% du volume du compartiment d'homogénéisation (1).

8. Procédé en continu de fabrication de microparticules ou de nanoparticules mettant en oeuvre le dispositif selon l'une quelconque des revendications 1 à 7, **caractérisé en ce que** l'on délivre simultanément dans le compartiment d'homogénéisation (1) par la première entrée (2) une phase organique comprenant une substance active, un polymère et un solvant et par la deuxième entrée (3) une phase aqueuse comprenant un surfactant et que l'on applique à l'ensemble rotor (11) / stator (12) une vitesse tangentielle de 1,5 m/s à 50 m/s de manière à simultanément former une émulsion desdites phases et extraire du solvant contenu dans la phase organique, de façon à obtenir une suspension de particules à partir de laquelle lesdites particules sont isolées.

9. Procédé selon la revendication 8, **caractérisé en ce que** l'on disperse radialement la phase organique au travers de perforations (10) disposées sur la deuxième entrée (2).

10. Procédé selon l'une des revendications 8 et 9 dans lequel on isole lesdites particules en évacuant ladite suspension par la sortie (5) du compartiment d'homogénéisation (1) dans un récipient tampon et que l'on effectue en continu une ultrafiltration de ladite suspension.

11. Procédé selon l'une des revendications 8 et 9 dans lequel on isole lesdites particules en évacuant ladite suspension par la sortie (5) du compartiment d'homogénéisation (1) dans un récipient tampon et que l'on effectue en continu une filtration de ladite suspension.

12. Procédé mettant en oeuvre le dispositif selon l'une quelconque des revendications 1 à 7 pour la fabrication en continu de microparticules ou de nanoparticules a partir au moins d'une phase aqueuse et d'une phase organique, ledit procédé comprenant l'introduction d'une phase organique et d'une phase aqueuse dans un compartiment d'homogénéisation (1) qui renferme un système mélangeur (4) comprenant un ensemble rotor (11)/stator (12), puis l'extraction d'une suspension de microparticules ou de nanoparticules au travers d'une sortie se situant sur un côté du compartiment d'homogénéisation (1);
**caractérisé en ce que**
a) ladite introduction de la phase aqueuse et de la phase organique se fait horizontalement,
b) on entraîne l'ensemble rotor (11)/stator (12) autour d'un axe horizontal,
c) on introduit la phase organique dans un espace qui se situe à l'intérieur du rotor (11) et à l'intérieur du stator (12),
d) on extrait ladite suspension à travers le côté supérieur du compartiment d'homogénéisation (1).

## Claims

1. Device for the continuous manufacture of microparticles or nanoparticles from at least one aqueous phase and one organic phase comprising a homogenization compartment in the form of a cylinder (1) which is defined by a tubular wall forming the casing of the said cylinder and by a first side wall and a second side wall which are positioned at each end of the said tubular wall the device additionally comprising a first inlet and a second inlet (2, 3) which pass through the said first side wall and which are suitable for respectively delivering an organic phase and an aqueous phase to the homogenization compartment (1), an outlet (5) suitable for removing a particle suspension from the homogenization compartment (1); the homogenization compartment (1) including a mixing system (4) comprising a rotor (11)/stator (12) system, in which
a) the said side walls are positioned along a vertical plane,
b) the axis of symmetry of the said cylinder is positioned horizontally,
c) the rotor (11) is mounted rotationally about the horizontal axis which passes through the said second side wall,
d) the said first inlet (2) is a hollow tube positioned in the extension of the axis of the rotor (11),
e) the homogenization compartment (1) exhibits a top side on which the said outlet (5) is situated,
**characterized in that** the said hollow tube comprises a final part lying inside the rotor and inside the stator.

2. Device according to Claim 1, **characterized in that** the rotor (11) and the stator (12) are cylindrical in shape.

3. Device according to Claim 5, **characterized in that** the rotor (11) and stator (12) comprise a row of teeth (13) and that the spacing (14) between the teeth (13) is from 1 to 4 mm.

4. Device according to any one of the preceding claims, **characterized in that** the first inlet (2) comprises perforations (10).

5. Device according to Claim 4, **characterized in that** the number of perforations (10) is from 1 to 20.

6. Device according to Claim 4 or 5, **characterized in that** the perforations (10) have a diameter from 0.01 mm to 1 mm.

7. Device according to any one of the preceding claims, **characterized in that** the dimensions of the rotor (11)/stator (12) system are such that the said system occupies 4% to 40% of the volume of the homogenization compartment (1).

8. Continuous process for the manufacture of microparticles or nanoparticles employing the device according to any one of Claims 1 to 7, **characterized in that** an organic phase comprising an active substance, a polymer and a solvent and an aqueous phase comprising a surfactant are simultaneously delivered, via the first inlet (2) and via the second inlet (3) respectively, to the homogenization compartment (1) and **in that** a tangential velocity of 1.5 m/s to 50 m/s is applied to the rotor (11)/stator (12) system so as simultaneously to form an emulsion of the said phases and to extract solvent present in the organic phase, so as to obtain a suspension of particles from which the said particles are isolated.

9. Process according to Claim 8, **characterized in that** the organic phase is dispersed radially through perforations (10) positioned on the second inlet (2).

10. Process according to either of Claims 8 and 9, in which the said particles are isolated by discharging the said suspension via the outlet (5) of the homogenization compartment (1) into a storage receptacle and that the said suspension is subjected to continuous ultrafiltration.

11. Process according to either of Claims 8 and 9, in which the said particles are isolated by discharging the said suspension via the outlet (5) of the homogenization compartment (1) into a storage receptacle and that the said suspension is subjected to continuous filtration.

12. Process employing the device according to any one of Claims 1 to 7 for the continuous manufacture of microparticles or nanoparticles from at least one aqueous phase and one organic phase, the said process comprising the introduction of an organic phase and an aqueous phase into a homogenization compartment (1) which includes a mixing system (4) comprising a rotor (11)/stator (12) system and then the removal of a suspension of microparticles or nanoparticles through an outlet lying on a side of the homogenization compartment (1);
**characterized in that**
a) the said introduction of the aqueous phase and of the organic phase takes place horizontally,
b) the rotor (11)/stator (12) system is driven about a horizontal axis,
c) the organic phase is introduced into a space which is situated inside the rotor (11) and inside the stator (12),
d) the said suspension is removed through the top side of the homogenization compartment (1).

## Patentansprüche

1. Vorrichtung zur kontinuierlichen Herstellung von Mikropartikeln oder von Nanopartikeln aus mindestens einer wässrigen Phase und einer organischen Phase, umfassend eine Homogenisierungskammer in Form eines Zylinders (1), die durch eine röhrenförmige Wand, welche die äußere Hülle des Zylinders bildet, sowie durch eine erste und eine zweite Seitenwand, welche an den beiden Enden der röhrenförmigen Wand angeordnet sind, begrenzt wird; die Vorrichtung umfasst weiterhin eine erste und eine zweite Eintrittsöffnung (2, 3), welche sich in der ersten Seitenwand befinden und welche dafür geeignet sind, der Homogenisierungskammer (1) jeweils eine organische Phase beziehungsweise eine wässrige Phase zuzuführen, sowie eine Austrittsöffnung (5), welche dafür geeignet ist, der Homogenisierungskammer (1) eine Partikelsuspension zu entnehmen; innerhalb der Homogenisierungskammer (1) befindet sich ein Mischsystem (4), welches eine Einheit aus Rotor (11) und Stator (12) umfasst, wobei
a) die Seitenwände in einer vertikalen Ebene angeordnet sind,
b) die Symmetrieachse des Zylinder horizontal angeordnet ist,
c) der Rotor (11) derart angebracht ist, dass er um eine horizontale Achse rotiert, welche durch die zweite Seitenwand führt,
d) es sich bei der ersten Eintrittsöffnung (2) um eine Kanüle handelt, die in der Verlängerung der Achse des Rotors (11) angeordnet ist,
e) die Homogenisierungskammer (1) eine Oberseite aufweist, auf welcher sich die Austrittsöffnung (5) befindet,
**dadurch gekennzeichnet, dass** die Kanüle ein Endstück umfasst, das sich innerhalb des Rotors und innerhalb des Stators befindet.

2. Vorrichtung gemäß Anspruch 1, **dadurch gekennzeichnet, dass** der Rotor (11) und der Stator (12) von zylindrischer Form sind.

3. Vorrichtung gemäß Anspruch 5, **dadurch gekennzeichnet, dass** der Rotor (11) und der Stator (12) eine Reihe von Zähnen (13) umfassen und dass der Freiraum (14) zwischen den Zähnen (13) 1 bis 4 mm beträgt.

4. Vorrichtung gemäß einem beliebigen der vorhergehenden Ansprüche, **dadurch gekennzeichnet, dass** die erste Eintrittsöffnung (2) Perforationen (10) aufweist.

5. Vorrichtung gemäß Anspruch 4, **dadurch gekennzeichnet, dass** die Perforationen (10) aus einer Lochzahl von 1 bis 20 bestehen.

6. Vorrichtung gemäß Anspruch 4 oder 5, **dadurch gekennzeichnet, dass** der Lochdurchmesser der Perforationen (10) zwischen 0,01 mm und 1 mm liegt.

7. Vorrichtung gemäß einem beliebigen der vorhergehenden Ansprüche, **dadurch gekennzeichnet, dass** die Einheit aus Rotor (11)/Stator (12) derart dimensioniert ist, dass das System zwischen 4 % und 40 % des Volumens der Homogenisierungskammer (1) einnimmt.

8. Kontinuierliches Verfahren zur Herstellung von Mikropartikeln oder von Nanopartikeln, bei welchem die Vorrichtung gemäß einem beliebigen der Ansprüche 1 bis 7 eingesetzt wird, **dadurch gekennzeichnet, dass** der Homogenisierungskammer (1) über die erste Eintrittsöffnung (2) eine organische Phase, die einen Wirkstoff, ein Polymer und ein Lösemittel umfasst, zugeführt wird, während ihr gleichzeitig über die zweite Eintrittsöffnung (3) eine wässrige Phase, die ein Tensid umfasst, zugeführt wird, und dass die Einheit aus Rotor (11)/Stator (12) mit einer Tangentialgeschwindigkeit von 1,5 m/s bis 50 m/s derart zur Anwendung kommt, dass gleichzeitig die Phasen eine Emulsion bilden und das Lösemittel, das in der organischen Phase enthalten ist, extrahiert wird, so dass eine Partikelsuspension erhalten wird, aus welcher die Partikel isoliert werden können.

9. Verfahren gemäß Anspruch 8, **dadurch gekennzeichnet, dass** die organische Phase durch die Perforationen (10), die sich an der zweiten Eintrittsöffnung (2) befindet, radial dispergiert wird.

10. Verfahren gemäß einem der Ansprüche 8 und 9, wobei die Partikel isoliert werden, indem die Suspension durch die Austrittsöffnung (5) der Homogenisierungskammer (1) in einen Zwischenbehälter abgelassen wird, wobei die Suspension in einem kontinuierlichen Verfahren einer Ultrafiltration unterzogen wird.

11. Verfahren gemäß einem der Ansprüche 8 und 9, wobei die Partikel isoliert werden, indem die Suspension durch die Austrittsöffnung (5) der Homogenisierungskammer (1) in einen Zwischenbehälter abgelassen wird, wobei die Suspension in einem kontinuierlichen Verfahren einer Filtration unterzogen wird.

12. Verfahren, bei welchem die Vorrichtung gemäß einem beliebigen der Ansprüche 1 bis 7 eingesetzt wird, um kontinuierlich Mikropartikel oder Nanopartikel aus mindestens einer wässrigen Phase und einer organischen Phase herzustellen, wobei das Verfahren das Einleiten einer organischen Phase und einer wässrigen Phase in eine Homogenisierungskammer (1) umfasst, wobei sich innerhalb letzterer ein Mischsystem (4) befindet, welches eine Einheit aus Rotor (11)/Stator (12) umfasst, woraufhin eine Suspension von Mikropartikeln oder von Nanopartikeln durch eine Ausgangsöffnung, die sich auf einer Seite der Homogenisierungskammer (1) befindet, entnommen wird;
**dadurch gekennzeichnet, dass**
a) das Einleiten der wässrigen Phase und der organischen Phase auf horizontale Weise erfolgt,
b) die Einheit aus Rotor (11)/Stator (12) um eine horizontale Achse herum angetrieben wird,
c) die organische Phase in einen Bereich eingeleitet wird, der sich innerhalb des Rotors (11) und innerhalb des Stators (12) befindet,
d) die Suspension über die Oberseite der Homogenisierungskammer (1) entnommen wird.
